# EUROPEAN PATENT APPLICATION

(11) **EP 3 103 503 A1**
(43) Date of publication of application: **14.12.2016**
(21) Application number: 15191116.1
(22) Date of filing: 22.10.2015
(51) Int. Cl.: A61M 37/00, A61N 2/06

(54) **A TYPE OF TATTOOING NEEDLE ASSEMBLY**

(30) Priority: 10.06.2015 CN 201520396152 U
(71) Applicant: Buddha Brand Tattoo Limited, Christchurch, Dorset BH23 1EF (GB)
(72) Inventor: Zhao, Juan, 422714 Xingning County (CN)
(74) Representative: chapman + co

(57) **Abstract**

A type of tattooing needle assembly, including a main shell 1, a needle component 3 and a needle aperture 4 within that main shell, whereby one end of the main shell connects with the needle aperture, the other end connecting with an end cap 5 having a through-hole; a needle guide wall, the aforementioned main shell includes the main shell connecting end which connects with the needle aperture and the tattooing needle connecting end which connects to a tattooing unit; six clamps 7 are located evenly around the external circumference on the round external surface of the aforementioned main shell connecting end interlocking with external clamp grooves 8 on the external circular surface of the needle aperture connecting end; the internal surface of the main shell and the needle aperture connecting end being connected via a positioning groove 10 and a positioning lug 11.

## Description

### Technical field

This invention relates to a type of tattooing or permanent make-up tool, in particular it is a type of tattooing needle assemblage.

### Technical background

Commonly encountered tattooing assemblies consist of a tattooing unit, a tattooing unit shank, a needle aperture and the tattooing needle. Before use, the operator must assemble these components. The tattooing unit shank connects with the tattoo unit, the tattooing needle passing through the hollow centre of the shank. The needle aperture is either connected to the shank or is integrated in the shank. The tip section of the tattooing needle relies on the needle aperture to act as a front mount and a guide component, the rear end of the tattoo needle connecting to a component within the tattooing unit that causes the back and forth motion, the component causing the back and forth motion which acts as the mount for the rear end of the tattooing needle also causes the backwards and forwards motion of the tattooing needle. For safety's sake, the tattooing needle is disposable, whilst disposable needle apertures and shanks are also widely used.

In current tattooing assemblages, the tattooing needle and needle aperture are installed separately, as such, when the operator changes the tattooing needle, they need to fit together these structures. In particular, when it comes to inserting the tattooing needle into the needle aperture, this requires a great deal of care, in order to avoid scraping the inner wall of the needle aperture with the tip of the needle causing damage to the needle tip which would then lose sharpness; apart from this, during the tattooing process, based on the complexity of the design, the operator needs to switch to tattooing needles with different specifications a number of times, which also requires selection of needle apertures with different specifications, however the current design whereby the tattooing needle and needle aperture are separate clearly results in a relatively time-consuming fitting process.

Patent application CN200410062070.6 revealed a type of removable disposable component that fits onto the shank, the needle of which was supported at one of its ends causing it to remain on the axis of the needle, whilst moving within the needle guide component formed by the disposable component, whereby the aforementioned needle extended at its other end beyond the needle shaped jet opening allowing the delivery of the ink, the aforementioned driving structure being an assembly of a number of components, which connect to the needle shaft thus causing the back and forth movement of the needle; the aforementioned driving structure being an assembly of a number of components including a driving unit and a connecting assemblage, the connecting assemblage connecting the driving assemblage to the needle shaft. At least one part of the connecting assemblage is located within the disposable component, thus allowing its removal from the shank in conjunction with the disposable component. With such a scheme, there are too many parts to the disposable component, making its fitting inconvenient; apart from this, none of the current tattooing needle assemblages have given consideration to the ease with which the tattooing needle assembly can be changed during actual usage. In addition to this, with the traditional spring return assemblages which rely on tensioned rubber or a spring, there is a certain amount of noise whilst these have a short useful life, the reason for this being that due to the strong tensioned rubber or traditional spring, jolting vibration occurs, and at the same time as the needle is driven to shake, mechanical noise results, which can affect operation of the machine by the tattooist.

### Summary of the Invention

In the light of the drawbacks of current technology, this invention provides a type of tattooing needle assembly, which reduces the number of component parts, the assembly and disassembly of which is more simple and convenient, whilst at the same time, it provides a new structural design, which achieves smooth backwards and forwards movement of the needle, reducing the level of noise, whilst satisfying technical requirements regarding ease of replacement of the tattooing needle, whilst also reducing the level of manual fatigue experienced by the tattooist.

The technical scheme of this invention is: A type of tattooing needle assembly, including a main shell, and needle component and a needle aperture within that main shell, whereby one end of the main shell connects with the needle aperture, the other end connecting with an end cap, there being a through-hole in that end cap; on one side within the needle aperture there is a needle guide wall, the needle component extending outside of the main shell through the through-hole, the other end fitting closely with the needle guide wall and extending beyond the needle aperture; the aforementioned main shell includes a main shell connecting end which connects with the needle aperture and the tattooing unit connecting end which connects to a tattooing unit; there being six clamps located evenly around the external circumference on the round external surface of the aforementioned main shell connecting end, there being external clamp grooves that correspond to the clamps on the external circular surface of the needle aperture connecting end thus connecting the aforementioned needle aperture and main body; there is an positioning groove on the face of the needle aperture connecting end that matches up with the main shell, there being a positioning lug on the internal surface of the main shell connecting end in a matching position; there are a pair of clips on the exterior of the hollow tubular shaft of the tattooing unit connecting end, the main shell being connected to the tattooing unit via these clips.

The aforementioned clips have a symmetrical shape which rotates around the axial cross-section of the hollow tubular shaft, the aforementioned shape consisting of two crescent shapes, the aforementioned symmetrical rotating shape rotating around the centre of the round cross-section, the intersection point between the large end sections of these crescent shapes and the round cross-section being symmetrical in terms of the round cross-section.

The aforementioned needle component includes a needle and a connecting assemblage connecting the needle with the main shell, wherein this allows the needle to pass backwards and forwards through the aforementioned through-hole.

The aforementioned tattooing needle assembly to which it is also possible to attach a protective needle sleeve, there being internal clamping grooves located on that protective needle sleeve that correspond to the main shell clamps.

The aforementioned connecting assemblage includes an upper magnetic ring and a lower magnetic ring fixed onto the main shell, there being an integrated mounting plate located within the internal cavity of the main shell, there being a through-hole on the same axis as the internal cavity of the main shell within that mounting plate, that through-hole allowing the free movement of the needle shaft; there is a needle holder located in the centre of that needle shaft, the aforementioned upper magnetic ring and end cap fitting over two sides of the needle holder, the lower magnetic ring fitting over the bottom section of the needle shaft, the internal cavity of the tubular shaft between the aforementioned end cap and mounting plate allowing backwards and forwards movement of the upper magnetic ring and lower magnetic ring along that axis; the bottom end of the upper magnetic ring and the top end of the lower magnetic ring having the same magnetic polarity, by which the upper magnetic ring is magnetically suspended above the lower magnetic ring.

The aforementioned upper magnetic ring and lower magnetic ring are medical/health care grade magnetic rings.

The advantages of this invention are that: the connected assembly of the main shell and the needle aperture in this scheme result in the simplification of the tattooing needle assembly structure, being beneficial in terms of reducing costs; installing clamps on the main shell not only allows the needle aperture to be locked in effectively, but also allows matching of other assemblages for fixing the tattooing needle assembly or convenient manual assembly of the tattooing needle assembly; the installation of clips on the main shell allows for easier separation or installation of the tattooing needle assembly from the tattooing unit, separation or installation of the tattooing needle assembly from the tattooing unit being achieved simply by their twisting to a certain angle; the backwards and forwards movement of the needle is due to the repulsion between the poles of like polarity of the two magnetic rings, this reducing the problems of friction with the internal wall of the main shell and noise when the magnetic rings move back and forth, this reducing operator manual fatigue due to vibration.

### Description of the Drawings

Figure 1 is a structural representation of the tattooing needle assembly to which this invention relates;
Figure 2 is a structural representation of the needle aperture to which this invention relates;
Figure 3 is a top view of the main shell of this invention;
Figure 4 is a cross-sectional structural representation of this invention;

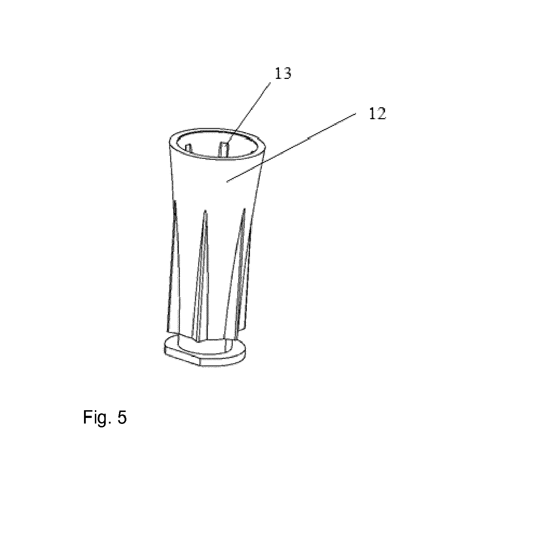

Figure 5 is a structural representation of the protective needle sleeve to which this invention relates.

In the diagrams, 1 is the main shell, 2 is the needle component, 3 is the needle aperture, 4 is the end cap, 5 is the main shell connecting end, 6 is the tattooing unit connecting end, 7 is a clamp, 8 is a clamp groove, 9 is the needle aperture connecting end, 10 is a positioning groove, 11 is a clip, 12 is the protective needle sleeve, 13 is a clamp internal groove, 14 is the upper magnetic ring, 15 is the lower magnetic ring, 16 is the mounting plate, 17 is the needle holder.

### Detailed Description

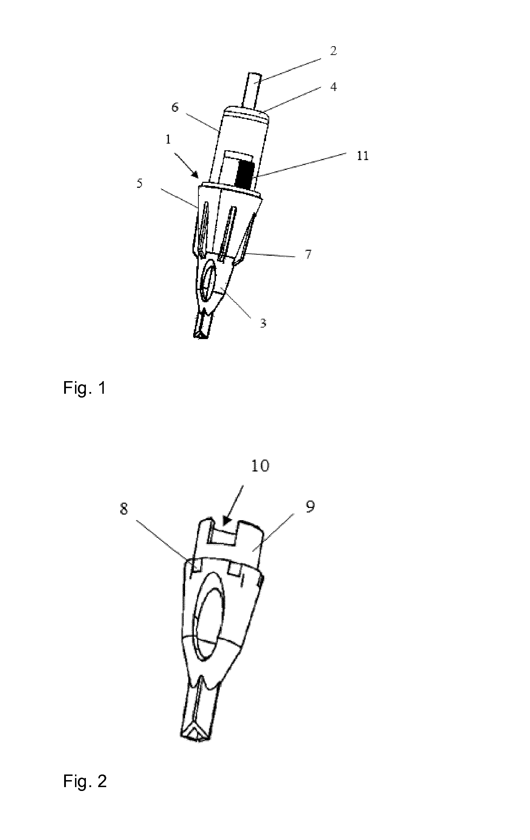

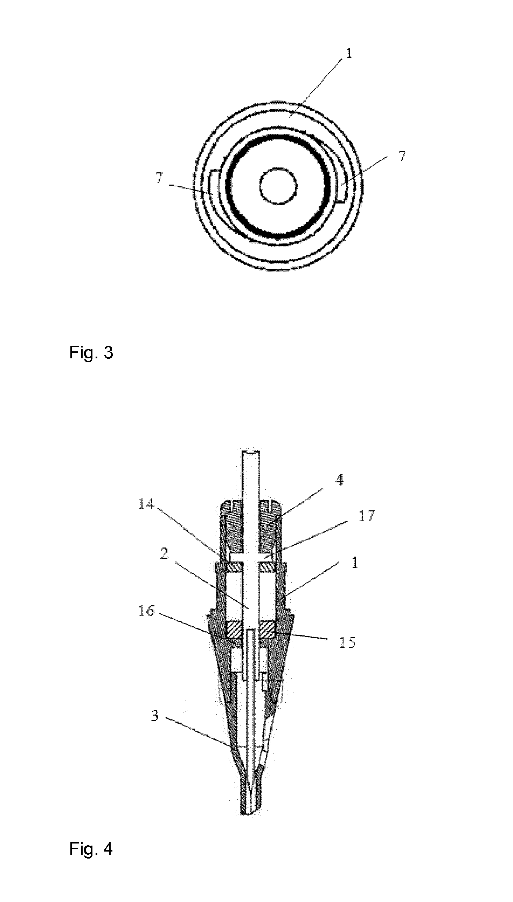

The following taken in conjunction with the appended diagrams provides a more detailed description of a specific implementation of this invention:
A type of tattooing needle assembly, as shown in figures 1-4, including a main shell 1, and a needle component 2 and a needle aperture 3 within that main shell 1, whereby one end of the main shell 1 connects with the needle aperture 3, the other end connecting with an end cap 4, there being a through-hole in that end cap 4, on one side within the needle aperture 3 there is a needle guide wall, the needle component 2 extending outside of the main shell 1 through the through-hole, the other end fitting closely with the needle guide wall and extending beyond the needle aperture 3; the aforementioned main shell 1 includes a main shell connecting end 5 which connects with the needle aperture 3 and the tattooing unit connecting end 6 which connects to a tattooing unit; there being six clamps 7 located evenly around the external circumference on the round external surface of the aforementioned main shell connecting end 5, there being external clamp grooves 8 that correspond to the clamps 7 on the external circular surface of the needle aperture connecting end 9 thus connecting the aforementioned needle aperture 3 and main body 1; there is a positioning groove 10 on the face of the needle aperture connecting end 9 that corresponds to the main shell 1, there being a positioning lug on the internal surface of the main shell connecting end 5 in a matching position; there are a pair of clips 11 on the exterior of the hollow tubular shaft of the tattooing unit connecting end 6, the main shell 1 being connected to the tattooing unit via these clips 11.

The aforementioned clips 11 have a symmetrical shape which rotates around the axial cross-section of the hollow tubular shaft, the aforementioned shape being one consisting of two crescent shapes, the aforementioned symmetrical rotating shape rotating around the centre of the round cross-section, the intersection point between the large end sections of these crescent shapes and the round cross-section, being symmetrical in terms of the round cross-section. The tattooing needle assembly clips together with the tattooing unit via these clips, rotation 20° clockwise allowing fixing of the tattooing needle assembly onto the tattooing unit, rotation anti-clockwise allowing separation of the tattooing needle assembly from the tattooing unit.

The aforementioned needle assembly 2 includes a needle and a connecting assemblage that allows connection of the needle and the main body 1, allowing the needle to move back and forth through the through-hole.

The aforementioned connecting assemblage includes an upper magnetic ring 14 and a lower magnetic ring 15 fixed onto the main shell 1, there being an integrated mounting plate 16 located within the internal cavity of the main shell 1, there being a through-hole on the same axis as the internal cavity of the main shell within that mounting plate 16, that through-hole allowing the free movement of the needle shaft; there is a needle holder 17 located in the central section of that needle shaft, the aforementioned upper magnetic ring 14 and end cap 4 fitting over two sides of the needle holder 17, the lower magnetic ring 15 fitting over the bottom section of the needle shaft, the internal cavity of the tubular shaft between the aforementioned end cap 4 and mounting plate 16 allowing backwards and forwards movement of the upper magnetic ring 14 and lower magnetic ring 15 along that axis; the bottom end of the upper magnetic ring 14 and the top end of the lower magnetic ring 15 having the same magnetic polarity, by which the upper magnetic ring is magnetically suspended above the lower magnetic ring.

The assemblage which causes the movable needle to bounce back and forth relies on the repulsion between two like polarity magnetic rings, thus resolving the problems of friction occurring with the main shell as the magnetic rings move back and forth and of noise. When used over a long time, the operator needs to hold the shank tight, the magnetic rings constructed of high strength permanent magnets creating a superficial magnetic field of 4800 Gauss. When the magnetic rings cause back and forth motion of the needle, the magnetic lines of force can penetrate the body to a depth of 6-9 cm, which is similar to the depth attained with traditional acupuncture, thus achieving the objective of providing a magnetic therapeutic effect to the hand. As everyone knows, the use of magnetic therapy (Magneto Therapy) or magnetic field therapy, the general name for methods relying on magnetic force or magnetic fields to treat diseases, involves placing the affected part within a variable magnetic field, with the intention of the magnetism generated by the magnetic field affecting bodily and skeletal tissue in order to achieve a non-invasive therapeutic objective. Magnetic therapy achieves the following functions: analgesia: the antiinflammatory effect is particularly pronounced, in particular it has a pronounced curative effect in the treatment of arthritis, strain injuries and other chronic diseases. For this reason, this scheme resolves the problems of strain injuries to the hand occurring due to the operator holding tightly onto the unit for long periods of time, in addition to having a magnetic health-promoting effect on the operator's hand.

It is also possible to attach a protective needle sleeve 12 to the aforementioned tattooing needle assembly, there being internal clamping grooves 13 located on that protective needle sleeve 12 that correspond to main shell 1 clamps 7.

The connected assembly of the main shell 1 and the needle aperture 3 in this scheme result in the simplification of the tattooing needle assembly, the combination of a needle aperture 3 positioning groove 10 which matches up with a positioning lug on the main shell 1 to achieve positioning is beneficial in terms of reducing costs; by installing clamps 7 on the main shell 1 and by installing external clamp grooves 8 on the needle aperture 3 that correspond to the clamps 7, allows rapid connection of the needle aperture 3 and the main body 1 ; the clamps 7 on the main shell 1 also help to prevent the hand from slipping when the tattooing needle assembly is being changed, allowing rapid manipulation of the tattooing needle assembly when carrying out replacement; in addition to this, the clamps 7 can also be used in combination with other assemblages to secure the tattoo needle assembly, making it suitable for automation; the clamps 7 may also be used for secure clipping on of the protective needle sleeve 12, thus providing effective protection to the needle point of the needle component 2. There are clips located on the main shell 1, which make disassembly and assembly of the tattooing needle assembly even more convenient, these only requiring twisting to a certain angle in order to disconnect or connect the tattooing needle assembly from the tattoo unit.

The aforementioned upper magnetic ring 14 and lower magnetic ring 15 are constructed from medical/health care grade magnetic rings, resulting in their possessing a kind of naturally generated force provided by Nature.

The contents of the above mentioned implementation and description are simply to provide a description of an optimal implementation of this invention, and where these do not depart from the spirit and scope of this invention, it would be possible to make many modifications or improvements to this invention, and these modifications and improvements shall all fall within the scope of protection claimed for this invention.

## Claims

1. A type of tattooing needle assembly, including a main shell, and needle component and a needle aperture within that main shell, whereby one end of the main shell connects with the needle aperture, the other end connecting with an end cap, there being a through-hole in that end cap; on one side within the needle aperture there is a needle guide wall, the needle component extending outside of the main shell through the through-hole, the other end fitting closely with the needle guide wall and extending beyond the needle aperture; the aforementioned main shell includes a main shell connecting end which connects with the needle aperture and the tattooing unit connecting end which connects to a tattooing unit; there being six clamps located evenly around the external circumference on the round external surface of the aforementioned main shell connecting end, there being external clamp grooves that correspond to the clamps on the external circular surface of the needle aperture connecting end thus connecting the aforementioned needle aperture and main body; there is a positioning groove on the face of the needle aperture connecting end that matches up with the main shell, there being a positioning lug on the internal surface of the main shell connecting end in a matching position; there are a pair of clips on the exterior of the hollow tubular shaft of the tattooing unit connecting end, the main shell being connected to the tattooing unit via these clips.

2. The tattooing needle assembly according to claim 1, whereby the aforementioned clips have a symmetrical shape which rotates around the axial cross-section of the hollow tubular shaft, the aforementioned shape being one consisting of two crescent shapes, the aforementioned symmetrical rotating shape rotating around the centre of the round cross-section, the intersection point between the large end sections of these crescent shapes and the round cross-section, being symmetrical in terms of the round cross-section.

3. The tattooing needle assembly according to claim 1, whereby the aforementioned needle component includes a needle and a connecting assemblage connecting the needle with the main shell, wherein this allows the needle to pass backwards and forwards through the aforementioned through-hole.

4. The tattooing needle assembly according to claim 1, whereby it is also possible to attach a protective needle sleeve to the tattooing needle assembly, there being internal clamping grooves located on that protective needle sleeve that correspond to the main shell clamps.

5. The tattooing needle assembly according to claim 3, whereby the aforementioned connecting assemblage includes an upper magnetic ring and a lower magnetic ring fixed onto the main shell, there being an integrated mounting plate located within the internal cavity of the main shell, there being a through-hole on the same axis as the internal cavity of the main shell within that mounting plate, that through-hole allowing the free movement of the needle shaft; there is a needle holder located in the centre of that needle shaft, the aforementioned upper magnetic ring and end cap fitting over two sides of the needle holder, the lower magnetic ring fitting over the bottom section of the needle shaft, the internal cavity of the tubular shaft between the aforementioned end cap and mounting plate allowing backwards and forwards movement of the upper magnetic ring and lower magnetic ring along that axis; the bottom end of the upper magnetic ring and the top end of the lower magnetic ring having the same magnetic polarity, by which the upper magnetic ring is magnetically suspended above the lower magnetic ring.

6. The tattooing needle assembly according to claim 5, whereby the aforementioned upper magnetic ring and lower magnetic ring are medical/health care grade magnetic rings.
